# EUROPEAN PATENT SPECIFICATION

(11) **EP 1 776 065 B1**
(45) Date of publication and mention of the grant of the patent: **15.02.2012**
(21) Application number: 05786506.5
(22) Date of filing: 12.08.2005
(51) Int. Cl.: A61F 2/00

(54) **SURGICAL PROSTHESIS HAVING BIODEGRADABLE AND NONBIODEGRADABLE REGIONS**
CHIRURGISCHE PROTHESE MIT BIOLOGISCH ABBAUBAREN UND NICHTABBAUBAREN REGIONEN
PROTHESE CHIRURGICALE PRESENTANT DES REGIONS BIODEGRADABLES ET NON BIODEGRADABLES

(30) Priority: 13.08.2004 US 601414 P; 28.10.2004 US 623524 P
(43) Date of publication of application: 25.04.2007
(73) Proprietor: Mast Biosurgery AG, 8002 Zürich (CH)
(72) Inventor: DEUSCH, Kai, CH-8002 Zurich (CH); CALHOUN, Christopher, J., Del Mar, California 92130 (US); MULLINS, Kenton, R., Huntington Beach, California 92648 (US)
(74) Representative: Potter Clarkson LLP
(86) International application number: PCT/US2005/028834
(87) International publication number: WO 2006/020922

(56) References cited:
- EP-A- 1 384 450
- WO-A-93/17635
- US-A- 5 686 090
- US-A- 2005 175 665

## Description

### BACKGROUND OF THE INVENTION

### 1. Field of the Invention

This invention relates to surgical prostheses for repairing abdominal hernias.

### 2. Description of Related Art

A hernia is defined as a defect in the strong or fascia layer of the abdominal wall which allows abdominal organs (e.g., intestine and/or omentum) to protrude. Once out of their normal position, these organs can become pinched or twisted. The most common hernia symptoms are abdominal pain, nausea, vomiting, and an abdominal mass or lump that may come and go. Hernias are commonly caused by previous surgical incisions, but can also occur without a previous surgery.

Treatment for hernias is surgical repair. There are no special exercises that can strengthen the tissues or any medications to take. Repair of the hernia is achieved by closing the defect in the strong or fascia layer of the abdominal wall. A special synthetic material called a mesh is commonly utilized in repairing the defect in order to add extra strength.

A conventional procedure for repairing a hernia involves making an incision over the site of the hernia, pushing the internal viscera back into the abdominal cavity and closing the opening by stitching or suturing one side firmly to the other. Another procedure involves making the incision, placing a piece of knitted mesh material over the hernial opening, holding or suturing the mesh material firmly in place, and closing the incision.

EP1 384 450 discloses a flat implant for surgery. The implant includes a flexible fabric comprising two sides, one of which is substantially closed, the other of which has a 3D microstructure permitting a growing-in of cells.

### SUMMARY OF THE INVENTION

According to the present invention there is provided a biodegradable surgical implant for implantation within a host as claimed in Claim 1.

A prosthesis for repairing a hernia in accordance with the present invention comprises an adhesion-resistant biodegradable region and an opposing tissue-ingrowth biodegradable region comprising a first and second layer of biodegradable material respectively, When the prosthesis is implanted into the patient, the adhesion-resistant biodegradable region covers a fascial defect of the hernia, and the tissue-ingrowth biodegradable region is located above the adhesion-resistant biodegradable region while being exposed substantially only to the host's subcutaneous tissue (e.g., fat) layer. This orientation allows the tissue-ingrowth biodegradable region to become firmly incorporated with the host's body tissue, The adhesion-resistant biodegradable region faces the internal organs and decreases the incidence of adhesions and/or bowel obstruction.

In accordance with one aspect of the present invention, the adhesion-resistant biodegradable region comprises a rate of biodegradation which is substantially greater than a rate of biodegradation of the tissue-ingrowth biodegradable region. According to another aspect of the present invention, the adhesion-resistant biodegradable region comprises a resorbable polymer composition which is different than a resorbable polymer composition of the tissue-ingrowth biodegradable region.

Also described herein is a process for repairing a soft tissue defect of a patient by surgically implanting any prosthesis of this invention adjacent the soft tissue defect. In one embodiment example of the process the adhesion-resistant biodegradable region and the tissue-ingrowth biodegradable region are both surgically attached to the fascia, whereas in another example the tissue-ingrowth biodegradable region is surgically attached to the fascia while the adhesion-resistant biodegradable region is attached to the tissue-ingrowth biodegradable region and optionally to the fascia.

### BRIEF DESCRIPTION OF THE DRAWINGS

FIG. 1 is a perspective view of an embodiment of a biodegradable surgical prosthesis in accordance with the present invention;
FIG. 2 is a cross-sectional view of an abdominal wall that has been repaired using an embodiment of the biodegradable surgical prosthesis of the present invention; and
FIG. 3 is a cross-sectional view of an abdominal wall that has been repaired using another embodiment of the biodegradable surgical prosthesis of the present invention,

### DETAILED DESCRIPTION OF THE INVENTION

Any feature or combination of features described herein are included within the scope of the present invention provided that the features included in any such combination come within the scope of the claims.

It should be noted that the drawings are in simplified form and are not to precise scale. In reference to the disclosure herein, for purposes of convenience and clarity only, directional terms, such as, top, bottom, left, right, up, down, over, above, below, beneath, rear, and front, are used with respect to the accompanying drawings. Such directional terms should not be construed to limit the scope of the invention in any manner. Although the disclosure herein refers to certain illustrated embodiments, it is to be understood that these embodiments are presented by way of example and not by way of limitation. The intent of the following detailed description, although discussing exemplary embodiments, is to be construed to cover all modifications, alternatives, and equivalents of the embodiments as may fall within the and scope of the invention as claimed.

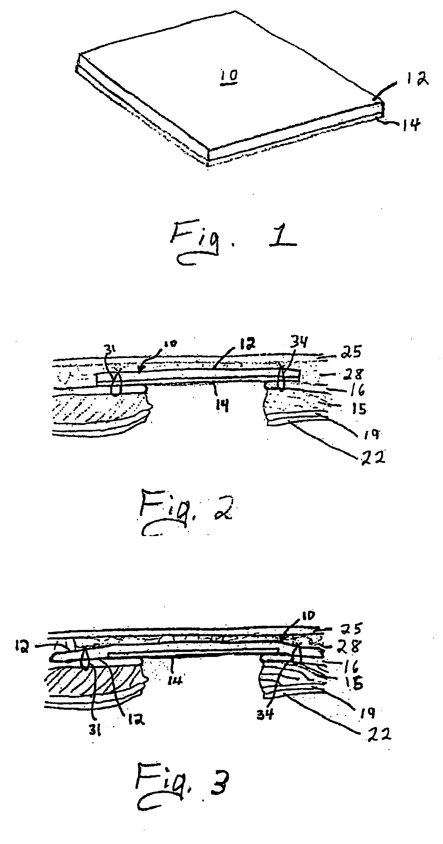

Referring more particularly to the drawings, a biodegradable surgical prosthesis 10 is shown in FIG. 1 comprising a tissue-ingrowth biodegradable region 12 and an opposing adhesion-resistant biodegradable region 14. The biodegradable surgical prosthesis 10 is constructed for use in the repair of soft tissue defects, such as soft tissue defects resulting from incisional and other hernias and soft tissue defects resulting from extirpative tumor surgery. The biodegradable surgical prosthesis 10 may also be used in cancer surgeries, such as surgeries involving sarcoma of the extremities where saving a limb is a goal. Other applications of the biodegradable surgical prosthesis 10 of the present invention may include laparoscopic or standard hernia repair in the groin area, umbilical hernia repair, paracolostomy hernia repair, femora hernia repair, lumbar hernia repair, and the repair of other abdominal wall defects, thoracic wall defects and diaphragmatic hernias and defects.

Each of the tissue-ingrowth biodegradable region 12 and the adhesion-resistant biodegradable region 14 can comprise, for example, a biodegradable, and more preferably bioresorbable, polyhydroxyacid material. According to certain strict definitions, biodegradable polymers, which may be used with the invention, require enzymes of microorganisms for hydrolytic or oxidative degradation, whereas bioresorbable polymers, which are presently preferred, degrade in the physiological environment with the by-products being eliminated or completely bioabsorbed. Generally, a polymer that loses its weight over time in the living body can be referred to as an absorbable, resorbable, bioabsorbable, or even biodegradable polymer. This terminology applies regardless of its degradation mode, in other words for both enzymatic and non-enzymatic hydrolysis. Biodegradable polymers, including resorbalbe polymers, can be classified on the basis of their origin as either naturally occurring or synthetic. Among synthetic resorbable polymers for implants, polyhydroxyacids occupy the main position. Non limiting examples of these each of which may individually or in combination be used to form all or part of the biodegradable prosthesis include poly(L-lactide), poly(glycolide) and polymers or copolymers based on L-lactide, L/DL-lactide, DL-lactide, glycolide, trimethyl carbonate, ε-caprolactone, dioxanone, and physical and chemical combinations thereof. Biodegradable polymer devices are eliminated from the body by hydrolytic degradation and subsequent metabolism after serving their intended purpose. In modified embodiments, part or all, in any combination, of the tissue-ingrowth region 12 can comprise or consist of a non-biodegradable polymer, such as, for example, one or more of (a) various thermoplastic resins that are polymers of, for example, propylene, (b) polymethacrylate, (c) polymethylmethacrylate (PMMA), or (d) combinations thereof.

According to an aspect of the present invention, the tissue-ingrowth biodegradable region 12 and the adhesion-resistant biodegradable region 14 may differ in both (A) surface appearance and (B) surface function. For example, the tissue-ingrowth biodegradable region 12 can be constructed with at least one of a surface topography (appearance) and a surface composition (function), either of which may facilitate strength, longevity and/or a substantial fibroblastic reaction in the host tissue relative to for example the anti-adhesion biodegradable region 14. On the other hand, the adhesion-resistant biodegradable region 14 can be constructed with at least one of a surface topography and a surface composition, either of which may facilitate, relative to the tissue-ingrowth biodegradable region 12, an anti-adhesive effect between the biodegradable surgical implant 10 and host tissues.

### A. Surface Topography (Appearance):

The tissue-ingrowth biodegradable region 12 can be formed to have an open, non-smooth and/or featured surface comprising, for example, alveoli distributed regularly or irregularly. In further embodiments, the tissue-ingrowth biodegradable region 12 can be formed to have, additionally or alternatively, an uneven (e.g., cracked, broken, roughened or flaked) surface which, as with the above-described surfaces, may cause tissue turbulence (e.g., potential tissue inflammation and/or scarring) between host tissues and the tissue-ingrowth biodegradable region 12.

Over time, with respect to the tissue-ingrowth biodegradable region 12, the patient's fibrous and collagenous tissue may substantially completely overgrow the tissue-ingrowth biodegradable region 12, growing over and affixing the tissue-ingrowth biodegradable region 12 to the tissue. In one implementation, the tissue-ingrowth biodegradable region 12 comprises a plurality of alveoli or apertures visible to the naked eye, through or over which the host tissue can grow and achieve substantial fixation.

As an example, pores may be formed into the tissue-ingrowth biodegradable region by punching or otherwise machining, or by using laser energy. Non-smooth surfaces may be formed, for example, by abrading the tissue-ingrowth biodegradable region 12 with a relatively course surface (e.g., having a 40 or, preferably, higher grit sandpaper-like surface) or, alternatively, non-smooth surfaces may be generated by bringing the tissue-ingrowth biodegradable region 12 up to its softening or melting temperature and imprinting it with a template (to use the same example, a sandpaper-like surface). The imprinting may occur, for example, during an initial formation process or at a subsequent time.

On the other hand, the adhesion-resistant non-porous biodegradable region 14 can be formed to have a closed, continuous, and/or smooth surface as described in claim 1. In an illustrative embodiment, at least a portion of the adhesion-resistant biodegradable region 14 is smooth comprising no protuberances, alveoli or vessel-permeable pores, so as to attenuate occurrences of adhesions between the tissue-ingrowth biodegradable region 12 and host tissues.

In a molding embodiment, one side of the press may be formed to generate any of the tissue-ingrowth biodegradable region surfaces discussed above and the other side of the press may be formed to generate an adhesion-resistant biodegradable region surface as discussed above. Additional features (e.g., roughening or forming apertures) may subsequently be added to further define the surface of, for example, the tissue-ingrowth biodegradable region. In an extrusion embodiment, one side of the output orifice may be formed (e.g. ribbed) to generate a tissue-ingrowth biodegradable region (wherein subsequent processing can further define the surface such as by adding transverse ribs/features and/or alveoli) and the other side of the orifice may be formed to generate an adhesion-resistant biodegradation region surface. In one embodiment, the adhesion-resistant biodegradable region is extruded to have a smooth surface and in another embodiment the adhesion-resistant biodegradable region is further processed (e.g., smoothed) after being extruded.

### B. Surface Composition (Function):

As presently embodied, the tissue-ingrowth biodegradable region 12 comprises a first material, and the adhesion-resistant biodegradable region 14 comprises a second material which is different from the first material. In modified embodiments, the tissue-ingrowth biodegradable region 12 and the adhesion-resistant biodegradable region 14 may comprise the same or substantially the same materials. In other embodiments, the tissue-ingrowth biodegradable region 12 and the adhesion-resistant biodegradable region 14 may comprise different materials resulting from, for example, an additive having been introduced to at least one of the tissue-ingrowth biodegradable region 12 and the adhesion-resistant biodegradable region 14.

The adhesion-resistant biodegradable region 14 can be formed to have any of the structures or dimensions disclosed in U.S. Patent No. 6,673,362, entitled BIODEGRADABLE BARRIER MICRO-MEMBRANES FOR ATTENUATION OF SCAR TISSUE DURING HEALING, and/or may be formed with or in combination with any of the materials described herein, preferably to facilitate tissue separation with attenuated (e.g., eliminated) adhesion.

According to an implementation of the present invention, the adhesion-resistant biodegradable region 14 is constructed to minimize an occurrence of adhesions of host tissues (e.g., internal body viscera) to the biodegradable surgical prosthesis 10. In being formed to be absorbable, the adhesion-resistant biodegradable region 14 should be sufficiently non-inflammatory while being absorbed so as not to cause adhesions itself. For example, it is believed that resorption into the body too quickly of the adhesion-resistant biodegradable region 14 may yield undesirable drops in local pH levels, thus possibly introducing/elevating, for example, local inflammation, discomfort and/or foreign antibody responses. As distinguished from the function(s) of the tissue-ingrowth biodegradable region 12, an object of the adhesion-resistant biodegradable region 14 can be to attenuate tissue turbulence and any accompanying inflammation (e.g., swelling).

In modified embodiments, the adhesion-resistant biodegradable region 14 and the tissue-ingrowth biodegradable region 12 of the biodegradable surgical prosthesis 10 may be formed of the same material or relatively less divergent materials, functionally speaking, and the adhesion-resistant biodegradable region 14 may be used in conjunction with an anti-inflammatory gel agent applied, for example, onto the adhesion-resistant biodegradable region 14 at a time of implantation of the biodegradable surgical prosthesis 10. According to other broad embodiments, the adhesion-resistant biodegradable region 14 and the tissue-ingrowth biodegradable region 12 may be formed of any materials or combinations of materials disclosed herein (including embodiments wherein the two regions share the same layer of material) or their substantial equivalents, and the adhesion-resistant biodegradable region 14 may be used in conjunction with an anti-inflammatory gel agent applied, for example, onto the adhesion-resistant biodegradable region 14 at a time of implantation of the biodegradable surgical prosthesis 10.

The tissue-ingrowth biodegradable region 12 can be formed of similar and/or different materials to those set forth above, to facilitate strength, longevity and/or direct post-surgical cell colonization via, for example, invoking a substantial fibroblastic reaction in the host tissue. In an illustrated embodiment, the tissue-ingrowth biodegradable region 12 is constructed to be substantially incorporated into the host tissue and/or to substantially increases the structural integrity of the biodegradable surgical prosthesis 10. Following implantation of the biodegradable surgical prosthesis 10, body tissues (e.g., subcutaneous tissue and/or the exterior fascia) commence to incorporate themselves into the tissue-ingrowth biodegradable region 12. While not wishing to be limited, it is believed that the body, upon sensing the presence of the tissue-ingrowth biodegradable region 12 of the present invention, is disposed to send out fibrous tissue which grows in, around and/or through and at least partially entwines itself with the tissue-ingrowth biodegradable region 12. In this manner, the biodegradable surgical prosthesis 10 can become securely attached to the host body tissue.

Regarding different materials, according to an aspect of the present invention, the tissue-ingrowth biodegradable region 12 can comprises a biodegradable (e.g., resorbalbe) polymer composition having one or more different characteristics than that or those of a biodegradable (e.g., resorbalbe) polymer composition of the adhesion-resistant biodegradable region 14. The different characteristics may include (1a) time or rate of biodegradation affected by additives, (1b) time or rate of biodegradation affected by polymer structures/compositions, (2) polymer composition affecting strength or structural integrity, and (3) ability to facilitate fibroblastic reaction.

### 1. Time or Rate of Biodegradation

The time or rate of biodegradation for the adhesion-resistant biodegradable region 14 may be substantially greater than the rate of biodegradation of the tissue-ingrowth biodegradable region 12. This rate differential may be effectuated through, for example, use of (a) additives and/or (b) polymer structures/compositions.

### a. Additives Affecting Biodegradation Time or Rate

In accordance with one implementation, the characteristic is a time or rate of biodegradation influenced by the incorporation of an additive to at least one of the tissue-ingrowth biodegradable region 12 and the adhesion-resistant biodegradable region 14. In accordance with one implementation of the present invention, a rate of biodegradation of the adhesion-resistant biodegradable region 14 is substantially greater than a rate of biodegradation of the tissue-ingrowth biodegradable region 12. To adjust the biodegradation rate, an accelerator or retardant can be provided in one or more of the tissue-ingrowth biodegradable region 12 and the adhesion-resistant biodegradable region 14.

The additive may comprise, in typical embodiments, one or more of (i) retardants for retarding a rate of biodegradation of a polymer when added to the polymer and (ii) accelerators for accelerating a rate of biodegradation of a polymer when added to the polymer. In accordance with an implementation of the present invention, retardants can be added to (e.g., incorporated into) the tissue-ingrowth biodegradable region 12 and/or accelerators can be added to (e.g., incorporated into) the adhesion-resistant biodegradable region 14.

Retardants of the present invention can include hydrophobic compounds (i.e., repelling, tending not to combine with, or incapable of dissolving in water), to decrease the rate of biodegradation. Agents which may serve as retardants in accordance with the present invention include non-water soluble polymers, e.g. high molecular weight methylcellulose and ethylcellulose, etc., and low water soluble organic compounds. Exemplary hydrophobic agents of an implementation of the invention may comprise compounds which have less than about 100µg/ml solubility in water at ambient temperature. According to a broad aspect of the invention, a retardant may include any matter which is hydrophobic, wherein one implementation includes particles, for example powders or granules, which are at least partially made up of hydrophobic polymers.

Accelerators of the present invention can include hydrophilic compounds (i.e., having an affinity for, readily absorbing, or dissolving in water), to increase the rate of biodegradation. The accelerators of the present invention may be physiologically inert, water soluble polymers, e.g. low molecular weight methyl cellulose or hydroxypropyl methyl cellulose; sugars, e.g. monosaccharides such as fructose and glucose, disaccharides such as lactose, sucrose, or polysaccharides such as cellulose, amylose, dextran, etc. Exemplary hydrophilic compounds of the invention may comprise components which have at least about 100µg/ml solubility in water at ambient temperature. According to a broad aspect of one implementation of the present invention, an accelerator may include any matter which is hydrophilic, wherein an implementation includes particles, for example powders or granules, which comprise hydrophilic polymers.

In an exemplary embodiment, the tissue-ingrowth biodegradable region 12 and the adhesion-resistant biodegradable region 14 both comprise resorbable compositions, and a resorption retarding agent (retardant) is provided in the tissue-ingrowth biodegradable region 12 so that the tissue-ingrowth biodegradable region 12 biodegrades at a relatively slow rate. In a modified embodiment, the retardant may also be provided in the adhesion-resistant biodegradable region 14 at, for example, the same or a lower concentration.

According to one implementation, the tissue-ingrowth biodegradable region 12 biodegrades at a relatively slow rate to provide ample time for host tissues to form over and into the space occupied by the tissue-ingrowth biodegradable region 12. For example, in accordance with one aspect the biodegradable surgical prosthesis 10 is biodegraded (e.g., resorbed) into a mammalian body within a period of about 24 months or longer from an initial implantation of the implant into the mammalian body. In one embodiment, the biodegradable surgical prosthesis 10 loses its mechanical strength within 18 months and, preferably, within 24 months and, more preferably, with a period of or greater than 36 or 48 months from the time of implantation.

### b. Polymer Structures/Compositions Affecting Biodegradation Times or Rates

In accordance with another implementation, the characteristic is a polymer composition of at least one of the tissue-ingrowth biodegradable region 12 and the adhesion-resistant biodegradable region 14. A rate of biodegradation of the tissue-ingrowth biodegradable region 12 can be relatively low and/or can be less than a rate of biodegradation of the adhesion-resistant biodegradable region 14. To obtain such a biodegradation rate, the tissue-ingrowth biodegradable region 12 can be formed, for example, with synthesized polymers that have hydrolytically stable linkages in the backbone relative to those of faster biodegrading polymers and/or to those of the adhesion-resistant biodegradable region 14. Common chemical functional groups suitable for formation of the tissue-ingrowth biodegradable region 12, in addition to those already described herein, can include esters, anhydrides, orthoesters, and amides. Depending on the chemical structure of the polymer backbone, degradation can occur by either surface or bulk erosion. Surface erosion can occur when the rate of erosion exceeds the rate of water penetration into the bulk of the polymer of either the tissue-ingrowth biodegradable region 12 or the adhesion-resistant biodegradable region 14. This type of degradation can be obtained, for example, in oly(anhydrides) and poly(ortho esters). The hydrolysis of bulk degrading bioresorbable polymers as described herein may typically proceed by loss of molecular weight at first, followed by loss of mass in a second stage. Generally, hydrolysis (including enzyme-mediated hydrolysis) is a preferred degradation mechanism for heterochain polymers in vivo. As an example, the degradation of poly(ε-caprolactone) and related polyesters such as poly(lactide) and its copolymers first involves non-enzymatic hydrolysis of ester linkages, autocatalyzed by the generation of carboxylic acid end groups, followed by the loss of mass.

In accordance with an aspect of the present invention, lengthening of the in vivo elimination time of bioresorbable polymers can be determined by one or more of the nature of the polymer chemical linkage, the solubility of the degradation products, the size (e.g., thickness), shape and density of the region or prosthesis, the drug or additive content, the molecular weight of the polymer, the extent of cross-linking of the polymer, and the implantation site. As an example, the size and form of the region or prosthesis can be used to control at least one of biodegradation time and rate. For instance, a smaller surface to mass ratio can be implemented to retard the rate of biodegradation of the tissue-ingrowth biodegradable region 12. A relatively thick construction of the tissue-ingrowth biodegradable region 12 is believed to decelerate the absorption time or rate thereof, compared to times or rates of absorption of thinner prostheses of the same material.

The tissue-ingrowth biodegradable region 12 of the present invention can have a uniform thickness greater than about 500 microns, or greater than about 1000 microns, and even greater than about 1500 or 3000 microns. A tissue-ingrowth biodegradable region 12 of a biodegradable surgical prosthesis 10 can be shaped at the time of surgery by bringing the material to its glass transition temperature, using heating iron, hot air, heated sponge or hot water bath methods. In certain embodiments, poly lactides which become somewhat rigid or brittle at greater thicknesses can be softened by formation with another polymer or copolymer, such as poly-ε-caprolactone. In modified embodiments, the poly lactides (or other materials forming part, most or substantially all of the tissue-ingrowth region 12) may alternatively or additionally be combined with one or more non-biodegradable polymers, such as, for example, one or more of (a) various thermoplastic resins that are polymers of, for example, propylene, (b) polymethacrylate, (c) polymethylmethacrylate (PMMA), or (d) combinations thereof. More generally, in examples wherein tissue-ingrowth biodegradable regions 12 are formed by polymers (e.g., homo and/or copolymers) derived from one or more cyclic esters, such as lactide (i.e., L, D, DL, or combinations thereof), ε-caprolactone, and glycolide, compositions can comprise about 1 to 99% ε-caprolactone, or 20 to 40% ε-caprolactone, with the remainder of the polymer comprising a lactide such as poly(L-lactide). In modified embodiments wherein tissue-ingrowth regions 12 are formed by polymers (e.g., homo and/or copolymers) derived from one or more cyclic esters and/or other materials, part or all of the tissue-ingrowth regions 12 can comprise or consist of one or more non-biodegradable polymers, such as, for example, one or more of (a) various thermoplastic resins that are polymers of, for example, propylene, (b) polymethacrylate, (c) polymethylmethacrylate (PMMA), or (d) combinations thereof.

In further embodiments, other softening polymers (e.g., having low glass transition temperatures) such as other lactones may be used with or as a substitute for ε-caprolactone. In still further embodiments, one or more non-biodegradable polymers, such as, for example, one or more of (a) various thermoplastic resins that are polymers of, for example, propylene, (b) polymethacrylate, (c) polymethylmethacrylate (PMMA), or (d) combinations thereof, may be used with or as a substitute for ε-caprolactone and/or other softening polymers or lactones.

A preferred form of polymer for the tissue-ingrowth biodegradable region 12 is semicrystalline poly(L-lactide), which can have a degradation time in the order of 3 to 5 years, as compared to poly(DL-lactide) which degrades in 12 to 16 months. Polyhydroxyacids degrade to monomeric acids and subsequently to carbon dioxide and water. These are removed from the body via respiratory routes and the kidneys (the Krebs cycle). Included among the polyesters of interest are polymers of D-lactic acid, L-lactic acid, racemic lactic acid, glycolic acid, polycaprolactone, and copolymers/combinations thereof. In modified embodiments, part or all, in any combination, of the polymer (e.g., polyester) or polymers can comprise or consist of a non-biodegradable polymer, such as, for example, one or more of (a) various thermoplastic resins that are polymers of, for example, propylene, (b) polymethacrylate, (c) polymethylmethacrylate (PMMA), or (d) combinations thereof.. By employing the L-lactate or D-lactate, for example, a slowly biodegrading polymer can be achieved for the tissue-ingrowth biodegradable region 12, while for the adhesion-resistant biodegradable region 14 degradation may be substantially enhanced with a racemate.

Copolymers of lactic and glycolic acid (poly(lactide-co-glycolides)) can be of particular interest, wherein the rate of biodegradation can be controlled by the ratio of glycolic to lactic acid. The degradation of lactic acid and/or glycolic acid polymers in biological medium occurs exclusively by a chemical mechanism of nonspecific hydrolysis. The products of this hydrolysis are metabolized and then eliminated by the human body. Chemical hydrolysis of the polymer is complete, whereby the more pronounced its amorphous character and the lower its molecular mass, the more rapidly it occurs. Accordingly, the tissue-ingrowth biodegradable region 12 may be formed, for example, using at least one polymer or copolymer having a less pronounced amorphous character and/or an increased molecular mass. Although the most rapidly degraded copolymer has roughly equal amounts of glycolic and lactic acid, either homopolymer is more resistant to degradation making it more suitable for formation of the tissue-ingrowth biodegradable region 12. Biodegradation rate or time thus may be decreased, for example, in the context of forming a tissue-ingrowth biodegradable region 12, by acting on the composition of the mixture and/or on the molecular mass of the polymer(s). The biocompatibility of the poly(lactide) and poly(lactide-co-glycolide) polymers makes them suitable supports for cellular growth and tissue regeneration in the context of the present invention. It should also be considered that, other things being equal, the ratio of glycolic acid to lactic acid may also affect the brittleness of the resulting biodegradable surgical prosthesis.

### 2. Polymer Composition Affecting Strength or Structural Integrity

Furthermore, the characteristic may be a strength, structural integrity, or a related parameter, wherein, for example, the effects of bulging, wrinkling and/or curling of the biodegradable surgical prosthesis 10 may be attenuated. Since the present invention seeks to allot a substantially greater proportion of the biodegradable surgical prosthesis' strength and structural integrity to the tissue-ingrowth biodegradable region 12, the focus of adding strength or structural integrity to the biodegradable surgical prosthesis 10 is directed on the tissue-ingrowth biodegradable region 12.

Properties which may be adjusted in accordance with the present invention to augment the mechanical performance of the tissue-ingrowth biodegradable region 12 are monomer selection, polymerization and process conditions, and the presence of additives (e.g. fillers). These properties, in turn, can be adjusted so as to influence one or more of the hydrophilicity, crystallinity, melt and glass transition temperatures, molecular weight, molecular weight distribution, end groups, sequence distribution (random versus block), and the presence of residual monomer or additives in the tissue-ingrowth biodegradable region 12. Furthermore, a portion or all of these properties in combination then can influence the rate of biodegradation of the tissue-ingrowth biodegradable region 12.

Lactide is the cyclic dimer of lactic acid, which exists in three stereoisomeric forms, L-lactide, naturally occurring isomer, D-lactide and meso-lactide, which contains an L-lactyl unit and a D-lactyl unit in the ring. Additionally, DL-lactide is an equimolar mixture of L- and D-lactides. In accordance with an implementation of the present invention, the tissue-ingrowth biodegradable region 12 comprises poly(L-lactide), which has been found to exhibit high tensile strength and low elongation and consequently to have a high modulus, rendering it more suitable than many amorphous polymers for load-bearing applications such as hernia mending and sutures. Poly(L-lactide) has a melting point around 170°C and glass transition temperature in the range of 55-60°C. Poly(DL-lactide) is an amorphous polymer (Tg 45-55°C), having a random distribution of both isomeric forms of lactic acid and lacking the ability to arrange into a crystalline organized structure. Poly(DL-lactide) has a lower tensile strength, slightly higher elongation and substantially more rapid degradation time, making it more attractive for use in, for example, construction of the adhesion-resistant biodegradable region 14. Poly(ε-caprolactone) is a ductile semicrystalline polymer, melting in the range of 54-64°C. The glass transition temperature of -60°C can be increased by copolymerisation with lactide, which also may enhance the biodegradation of the polymer. In modified embodiments, one or more non-biodegradable polymers, such as, for example, one or more of (a) various thermoplastic resins that are polymers of, for example, propylene, (b) polymethacrylate, (c) polymethylmethacrylate (PMMA), or (d) combinations thereof, may be combined with the poly(ε-caprolactone).
The tissue-ingrowth biodegradable region 12 of a biodegradable surgical prosthesis 10 in accordance with an aspect of the present invention can be manufactured of biodegradable polymers by using one polymer or a polymer alloy. The biodegradable surgical prosthesis 10 can be strengthened by reinforcing the material with fibers manufactured from a resorbable polymer or of a polymer alloy, or with biodegradable glass fibers, such as β-tricalsiumphosphate fibers, bio-glass fibers or CaM fibers, as described in, e.g., publication EP146398, the entire disclosure of which is incorporated herein by reference. In modified embodiments, the surgical prosthesis 10 can be modified (e.g., strengthened) by including (e.g., for reinforcement) fibers or other elements manufactured from or with, in part or entirely, non-biodegradable polymers, such as, for example, one or more of (a) various thermoplastic resins that are polymers of, for example, propylene, (b) polymethacrylate, (c) polymethylmethacrylate (PMMA), or (d) combinations thereof.

The tissue-ingrowth biodegradable region 12 according to another aspect of the present invention can further, or alternatively, comprise or consist of at least one outer layer, which is a surface layer that improves the toughness of the implant and/or operates as a hydrolysis barrier. Moreover, an interior of the biodegradable surgical prosthesis 10 may additionally or alternatively comprise or consist of a stiffer and/or stronger layer or core. To prepare an example of such an embodiment, the biodegradable surgical prosthesis can be coated (e.g., brush, spray, bond, or dip coated) with an outer layer having different chemical and mechanical properties (e.g., hydrolysis and/or strength retention) than the core of the region or prosthesis. In one such case, an outer layer having greater resistance to hydrolysis than the biodegradable surgical prostheses' strength-enhanced core can be used, enabling the prosthesis (after insertion in a patient) to retain its strength and biodegrade over a longer period of time than it would have without such an outer coating or enhanced interior.

### 3. Ability to facilitate Fibroblastic Reaction

According to another implementation of the present invention, the characteristic may comprise an ability to facilitate a substantial fibroblastic reaction in the host tissue. The tissue-ingrowth biodegradable region 12 can be constructed to facilitate a fibroblastic reaction, while the adhesion-resistant biodegradable region 14 preferably does not cause a fibroblastic reaction. The facilitation by the tissue-ingrowth biodegradable region 12 of a fibroblastic reaction can be based on one or more of the above-discussed characteristics (e.g., time or rate of biodegradation affected by additives, time or rate of biodegradation affected by polymer structures/compositions, and polymer composition affecting strength or structural integrity), since the biodegradable surgical prosthesis 10 will need to maintain its structure long enough for reacting tissues to take a firm hold.

In one embodiment, the tissue-ingrowth biodegradable region 12 of the present invention can comprise or consist of at least one outer layer, which is a tissue ingrowth promoter. In another embodiment, all or substantially all of the biodegradable surgical prosthesis 10, except for the adhesion-resistant biodegradable region 14, comprises a tissue ingrowth promoter.

When applied to a roughened tissue-ingrowth biodegradable region 12 comprising, for example, at least one of protuberances, alveoli and pores, the biodegradable surgical implant 10 can provide interstitial space for the host body tissue to enter by ingrowth. Tissue ingrowth promoters can render the interstitial space conducive to the ingrowth therein of body tissue by providing chemically and/or physically improved surface characteristics.

In accordance with one aspect of the present invention, the tissue-ingrowth biodegradable region 12 may comprises a substance for cellular control, such as at least one of a chemotactic substance for influencing cell migration, an inhibitory substance for influencing cell-migration, a mitogenic growth factor for influencing cell proliferation, a growth factor for influencing cell differentiation, and factors which promote neoangiogenesis (formation of new blood vessels).

In particular implementations, one or several growth promoting factors can be introduced into or onto the tissue-ingrowth biodegradable region 12, such as fibroblast growth factor, epidermal growth factor, platelet derived growth factor, macrophage derived growth factor, alveolar derived growth factor, monocyte derived growth factor, magainin, and so forth.

Furthermore, one or more medico-surgically useful substances may be incorporated into or onto the tissue-ingrowth biodegradable region 12, such as those which accelerate or beneficially modify a growth or healing process. For example, the tissue-ingrowth biodegradable region 12 can carry (e.g., via mixing during formation, implanting, or coating) one or more therapeutic agents chosen for one or more of antimicrobial properties, capabilities for promoting repair or reconstruction and/or new tissue, growth and/or for specific indications.

Antimicrobial agents such as broad spectrum antibiotics (gentamicin sulphate, erythromycin or derivatized glycopeptides) can be carried (e.g., via mixing during formation, implanting or coating) to aid in combating clinical and sub-clinical infections in a tissue repair site thus facilitating ingrowth of host tissues onto and/or into the tissue-ingrowth biodegradable region 12, As an example, one or more of the above additives may be incorporated into the polymer of the tissue-ingrowth biodegradable region 12 itself prior to forming the tissue-ingrowth biodegradable region 12 as part of the biodegradable surgical prosthesis 10, for example, by addition to the polymer in suitable amounts so that at the conclusion of the polymeric particle manufacturing process, the material of the tissue-ingrowth biodegradable region 12 will contain a predetermined amount of one or more of such substances which for example will be released gradually as the polymer is biodegraded.

As shown in FIG, 2, the biodegradable surgical prosthesis 10 can be used to facilitate repair of, for example, a hernia in the ventral region of a body. FIG. 3 shows an implanted biodegradable surgical prosthesis 10 having both an adhesion-resistant biodegradable region 14 and a tissue-ingrowth biodegradable region 12 partially disposed on one side and having a tissue-ingrowth biodegradable region 12 disposed on a second side of the biodegradable surgical prosthesis 10. The abdominal wall includes muscle 15 enclosed and held in place by an exterior fascia 16 and an interior fascia 19. An interior layer, called the peritoneum 22, covers the interior side of the interior fascia 19. The peritoneum 22 is a softer, more pliable layer of tissue that forms a sack-like enclosure for the intestines and other internal viscera. A layer of skin 25 end a layer of subcutaneous fat 28 cover the exterior fascia 16.

Surgical repair of a soft tissue defect (e.g., a hernia) can be performed by using, for examples, conventional techniques or advanced laparoscopic methods to close substantially all of a soft tissue defect. According to one implementation, an incision can be made through the skin 25 and subcutaneous fat 28, after which the skin 25 and fat 28 can be peeled back followed by any protruding internal viscera (not shown) being positioned internal to the hernia. In certain implementations, an incision can be made in the peritoneum 22 followed by insertion of the biodegradable surgical prosthesis 10 into the hernia opening so that the biodegradable surgical prosthesis 10 is centrally located in the hernia opening. One or both the tissue-ingrowth biodegradable region 12 and the adhesion-resistant biodegradable region 14 may be attached by, e.g., suturing to the same layer of the abdominal wall, e.g., the relatively-strong exterior fascia 16. Alternatively, the adhesion-resistant biodegradable region 14 may be attached to another member, such as the interior fascia 19 and/or the peritoneum 22. In FIG. 3, the tissue-ingrowth biodegradable region 12 is surgically attached to the exterior fascia 16 while the adhesion-resistant biodegradable region is attached to the tissue-ingrowth biodegradable region 12 and/or optionally to the exterior fascia 16 using, e.g., heat bonding, suturing, and/or other affixation protocols disclosed herein or their substantial equivalents. Those possessing skill in the art will recognize that other methods of sizing/modifying/orientating/attaching a biodegradable surgical prosthesis 10 of this invention may be implemented according to the context of the particular surgical procedure.

The size of the biodegradable surgical prosthesis 10 typically will be determined by the size of the defect. Use of the biodegradable surgical prosthesis 10 in a tension-free closure may be associated with less pain and less incidence of post surgical fluid accumulation. Exemplary sutures 31 and 34 may be implemented as shown to at least partially secure the biodegradable surgical prosthesis to the abdominal wall structure. The sutures 31 and 34 can be preferably implemented so that no lateral tension is exerted on the exterior fascia 16 and/or muscle 15. When disrupted, the skin 25 and fat 28 may be returned to their normal positions, with for example the incisional edges of the skin 25 and fat 28 being secured to one another using suitable means such as subsurface sutures.

In modified embodiments of the present invention, one or both of the tissue-ingrowth biodegradable region 12 and the adhesion-resistant biodegradable region 14 of the biodegradable surgical prosthesis 10, can be heat bonded (or in a modified embodiment, otherwise attached, such as by suturing). Heat bonding may be achieved, for example, with a bipolar electro-cautery device, ultrasonicly welding, or similar sealing between the tissue-ingrowth biodegradable region 12 and the adhesion-resistant biodegradable region 14 and/or directly to surrounding tissues. Such a device can be used to heat the biodegradable surgical prosthesis 10 at various locations, such as at edges and/or at points in the middle, at least above its glass transition temperature, and preferably above its softening point temperature. The material is heated, e.g., along with adjacent tissue, such that the two components bond together at their interface. The heat bonding may also be used initially, for example, to secure the tissue-ingrowth biodegradable region 12 to the adhesion-resistant biodegradable region 14. Since the tissue-ingrowth biodegradable region 12 serves more of a load-bearing function, a few typical embodiments may exclude heat-bonding as the sole means for securing this region to host tissues. In other embodiments, the technique of heat bonding the biodegradable surgical prosthesis 10 to itself or body tissue may be combined with another attachment method for enhanced anchoring. For example, the biodegradable surgical prosthesis 10 may be temporarily affixed in position using two or more points of heat bonding using an electro-cautery device, and sutures, staples or glue can subsequently (or in other embodiments, alternatively) be added to secure the biodegradable surgical prosthesis 10 into place.

The tissue-ingrowth biodegradable region 12 and the adhesion-resistant biodegradable region 14 may be arranged to form more than one layer or substantially one layer, or the regions may both belong to a single, integrally formed layer. For example, the tissue-ingrowth biodegradable region 12 and the opposing adhesion-resistant biodegradable region 14 may be arranged in two layers, wherein one of the regions is disposed on top of, and opposite to, the other region.

In one embodiment, the tissue-ingrowth biodegradable region 12 and the adhesion-resistant biodegradable region 14 may be combined on a single side of the biodegradable surgical prosthesis 10 in, for example, substantially one layer, wherein the regions are adjacent each other on one side of the biodegradable surgical prosthesis 10. As a slight deviation, a biodegradable surgical prosthesis having a tissue-ingrowth biodegradable region on at least one (and preferably, both) side(s) thereof may be manufactured using any of the techniques described herein and, subsequently, an adhesion-resistant biodegradable region may be formed on, e.g., one side, by smoothing, filling, or otherwise processing an area of the tissue-ingrowth biodegradable region with a suitable material as disclosed herein or technique (e.g., coating or filling with a liquid or flowable polymer composition, and/or mechanically smoothing) to thereby form an adhesion-resistant biodegradable region having adhesion-resistant properties relative to those of the tissue-ingrowth biodegradable region.

Similarly, as depicted in FIG. 3, a patch of adhesion-resistant biodegradable region 14 may be sized and affixed (e.g., heat bonded, such as with a bipolar electro-cautery device, ultrasonicly welded, or similarly affixed) at a time of implantation directly to at least one of the tissue-ingrowth biodegradable region 12 and surrounding host tissues. In modified embodiments, the affixing may be accomplished using, for example, press or adhesive bonding, or sutures. In further embodiments, at least part of the affixing may occur at a time of manufacture of the biodegradable surgical prosthesis 10 before packaging. The patch of adhesion-resistant biodegradable region 14 alternatively may be partially affixed (e.g., using techniques enumerated in this paragraph) at, for example, a non-perimeter or central area thereof to an area (e.g., a non-perimeter or central area) of the tissue-ingrowth biodegradable region 12, so that a surgeon can trim the adhesion-resistant biodegradable region 14 (and/or the tissue-ingrowth biodegradable region 12) at a time of implantation while the adhesion-resistant biodegradable implant 14 is affixed to the tissue-ingrowth biodegradable region 12. For instance, a tissue-ingrowth biodegradable region 12 may substantially surround an adhesion-resistant biodegradable region 14 on one side of the biodegradable surgical prosthesis 10, and only a tissue-ingrowth biodegradable region 12 may be formed on the other side of the biodegradable surgical prosthesis 10. In such an implementation, the adhesion-resistant biodegradable region 14 of the biodegradable surgical prosthesis 10 can be sized and shaped so as to substantially cover any opening created by the soft tissue defect, with the tissue-ingrowth biodegradable regions 12 facilitating surgical attachment to, and incorporation into, the host tissue on at least one side of, and, preferably, on both sides of, the biodegradable surgical prosthesis 10.

In modified embodiments, the tissue-ingrowth biodegradable region 12 and/or the adhesion-resistant biodegradable region 14 on a given surface or surfaces of the biodegradable surgical prosthesis 10 each may be of any size or shape suited to fit the particular soft tissue defect. For example, either of the tissue-ingrowth biodegradable region 12 and/or the adhesion-resistant biodegradable region 14 on a given surface of the biodegradable surgical prosthesis 10 may have shapes of ovals, rectangles and various complex or other shapes wherein, for each such implementation, the two regions may have essentially the same, or different, proportions and/or dimensions relative to one another.

In general, various techniques may be employed to produce the biodegradable surgical prosthesis 10, which typically has one or two layers defining the tissue-ingrowth biodegradable region 12 and the adhesion-resistant biodegradable region 14. Useful techniques include solvent evaporation methods, phase separation methods, interfacial methods, extrusion methods, molding methods, injection molding methods, heat press methods and the like as known to those skilled in the art. The tissue-ingrowth biodegradable region 12 and the adhesion-resistant biodegradable region 14 may comprise two distinct layers or may be integrally formed together as one layer.

An exemplary process for making a biodegradable surgical prosthesis of the present invention having an adhesion-resistant biodegradable region, and a tissue-ingrowth biodegradable region with an additive, includes the steps of (a) forming a polymer layer to define the anti-adhesion biodegradable region such as described in U.S. Patent No. 6,673,362; (b) providing a water hydrolysable polymer; (c) forming the hydrolysable polymer into an implantable solid portion; and (d) attaching the polymer layer to the implantable solid portion whereby the solid portion defines a tissue-ingrowth biodegradable region. The step of forming the hydrolysable polymer into an implantable solid portion can comprise adding a retardant to the hydrolysable polymer to form a mixture, followed by forming a layer from the mixture and subsequently drying and purifying the layer to form the implantable solid portion. The tissue-ingrowth biodegradable region 12 and the adhesion-resistant biodegradable region 14 may be partially or substantially entirely formed or joined together. Joining can be achieved by mechanical methods, such as by suturing or by the use of metal clips, for example, hemoclips, or by other methods, such as chemical or heat bonding.

The above-described embodiments have been provided by way of example, and the present invention is not limited to these examples. Multiple variations and modification to the disclosed embodiments will occur, to the extent not mutually exclusive, to those skilled in the art upon consideration of the foregoing description. Additionally, other combinations, omissions, substitutions and modifications will be apparent to the skilled artisan in view of the disclosure herein. Accordingly, the present invention is not intended to be limited by the disclosed embodiments.

## Claims

1. A biodegradable surgical implant (10) for implantation in a host, comprising a substantially planar barrier membrane of resorbable polymer base material having
a tissue-ingrowth biodegradable side (12) and
an adhesion-resistant biodegradable side (14),
the tissue-ingrowth biodegradable side (12) and the adhesion-resistant biodegradable side (14) comprising a second and a first layer of biodegradable material, respectively, and differing in both surface appearance and surface function, **characterised in that**
the tissue-ingrowth biodegradable side and the adhesion-resistant biodegradable side comprise a non-porous biodegradable material.

2. The biodegradable surgical implant (10) as set forth in claim 1, wherein the tissue-ingrowth biodegradable side (12) and the adhesion-resistant biodegradable side (14) are formed of substantially the same material.

3. The biodegradable surgical implant (10) as set forth in claim 2, wherein one of the tissue-ingrowth biodegradable side (12) and the adhesion-resistant biodegradable side (14) comprises an additive.

4. The biodegradable surgical implant (10) as set forth in claim 3, wherein the additive enhances a rate of biodegradation of the adhesion-resistant biodegradable side (14) relative to that of the tissue-ingrowth biodegradable side (12).

5. The biodegradable surgical implant (10) as set forth in claim 3, wherein the additive reduces a rate of biodegradation of the tissue-ingrowth biodegradable side (12) relative to that of the adhesion-resistant biodegradable side (14).

6. The biodegradable surgical implant (10) as set forth in claim 1, wherein one of the tissue-ingrowth biodegradable side (12) and the adhesion-resistant biodegradable side (14) comprises a first composition affecting the side's strength or structural integrity relative to a second composition of the other of the tissue-ingrowth biodegradable side and the adhesion-resistant biodegradable side.

7. The biodegradable surgical implant (10) as set forth in claim 6, wherein the first composition comprises a first polymer composition and the second composition comprises a second polymer composition that is different from the first polymer composition.

8. The biodegradable surgical implant (10) as set forth in claim 6, wherein the first composition provides the tissue-ingrowth biodegradable side (12) with a greater strength than a strength that the tissue-ingrowth biodegradable side (12) would have if it were formed of the second composition.

9. The biodegradable surgical implant (10) as set forth in claim 8, wherein the first composition comprises strengthening and reinforcing fibers.

10. The biodegradable surgical implant (10) as set forth in claim 8, wherein the first composition comprises a first polymer composition and the second composition comprises a second polymer composition that is different from the first polymer composition.

11. The biodegradable surgical implant (10) as set forth in claim 2, wherein:
the tissue-ingrowth biodegradable side (12) comprises a first composition;
the adhesion-resistant biodegradable side (14) comprises a second composition; and
the adhesion-resistant biodegradable side (14) comprises a resistance to adhesion that is greater than a resistance to adhesion that would be provided by the adhesion-resistance biodegradable side (14) if formed of the first composition.

12. The biodegradable surgical implant (10) as set forth in claim 11, wherein the first composition comprises a first polymer composition and the second composition comprises a second polymer composition that is different from the first polymer composition.

13. The biodegradable surgical implant (10) as set forth in claim 1, wherein:
the tissue-ingrowth biodegradable side (12) comprises a first composition;
the adhesion-resistant biodegradable side (14) comprises a second composition; and
the adhesion-resistant biodegradable side (14) comprises a resistance to adhesion that is greater than a resistance to adhesion that would be provided by the adhesion-resistance biodegradable side (14) if formed of the first composition.

14. The biodegradable surgical implant (10) as set forth in claim 13, wherein the first composition comprises a first polymer composition and the second composition comprises a second polymer composition that is different from the first polymer composition.

15. The biodegradable surgical implant (10) as set forth in claim 1, wherein:
the biodegradable surgical implant contains a single layer of resorbable polymer base material having a substantially uniform composition; and
a thickness of the single layer of resorbable polymer base material is greater than about 500 microns,

16. The biodegradable surgical implant (10) as set forth in claim 15, wherein the single layer of resorbable polymer base material comprises a material selected from the group consisting of a poly-lactide polymer and a copolymer of two or more poly-lactides.

17. The biodegradable surgical implant (10) as set forth in claim 15, wherein the resorbable polymer base material is poly (L-lactide-co-D,L-lactide).

18. The device as set forth in claim 15, wherein the single layer of resorbable polymer base material is not fluid permeable.

19. The biodegradable surgical implant (10) as set forth in claim 1, wherein only one side of the biodegradable surgical implant is impregnated with at least one of an anti-bone agent, a chemotactic substance for influencing cell-migration, an inhibitory substance for influencing cell-migration, a mitogenic growth factor for influencing cell proliferation, a growth factor for influencing cell differentiation, and factors which promote angiogenesis.

20. The biodegradable surgical implant (10) as set forth in claim 1, wherein the biodegradable surgical implant is sealed in a sterile packaging.

21. The biodegradable surgical implant (10) as set forth in claim 1, wherein the biodegradable surgical implant prevents scar formation.

22. The biodegradable surgical implant (10) as set forth in claim 1, wherein the biodegradable surgical implant prevents tissue adhesion.

23. The biodegradable surgical implant (10) as set forth in claim 1, wherein the biodegradable surgical implant comprises a poly-lactide polymer and a copolymer of at least two poly-lactides.

24. The biodegradable surgical implant (10) as set forth in claim 1, wherein the biodegradable surgical implant is impermeable to fluid.

25. The biodegradable surgical implant (10) as set forth in claim 1, wherein the tissue-ingrowth biodegradable side (12) and the adhesion-resistant biodegradable side (14) are formed of the same material.

26. The biodegradable surgical implant (10) as set forth in claim 1, wherein the biodegradable surgical implant contains a single layer of resorbable polymer that forms both the tissue-ingrowth biodegradable side (12) and the adhesion-resistant biodegradable side (14).

27. The biodegradable surgical implant (10) as set forth in claim 1, wherein:
the biodegradable surgical implant contains a single layer of resorbable polymer base material;
the tissue-ingrowth biodegradable side (12) is integrally formed with the resorbable polymer base material; and
the adhesion-resistant biodegradable side (14) is integrally formed with the resorbable polymer base material.

28. The biodegradable surgical implant (10) as set forth in claim 1, wherein the adhesion-resistant biodegradable side (14) and the tissue-ingrowth biodegradable side (12) comprise different materials.

29. The biodegradable surgical implant (10) as set forth in claim 1, wherein the tissue-ingrowth biodegradable side (12) is constructed with one or more of (a) a surface appearance in the form of a surface topography and (2) a surface function in the form of a surface composition, which differs from that of the anti-adhesion biodegradable side and which facilitates one or more of strength, longevity and a substantial fibroblastic reaction in tissue of the host relative to the adhesion-resistant biodegradable side (14).

30. The biodegradable surgical implant (10) as set forth in claim 29, wherein the tissue-ingrowth biodegradable side (12) is constructed with a surface topography, which differs from that of the anti-adhesion biodegradable side and which facilitates one or more of strength, longevity and a substantial fibroblastic reaction in tissue of the host relative to the adhesion-resistant biodegradable side (14).

31. The biodegradable surgical implant (10) as set forth in claim 30, wherein the tissue-ingrowth biodegradable side (12) and the adhesion-resistant biodegradable side (14) are formed of substantially the same material.

32. The biodegradable surgical implant (10) as set forth in claim 30, wherein the tissue-ingrowth biodegradable side (12) and the adhesion-resistant biodegradable side (14) are formed of the same material.

33. The biodegradable surgical implant (10) as set forth in claim 30, wherein the biodegradable surgical implant contains a single layer of resorbable polymer that forms both the tissue-ingrowth biodegradable side (12) and the adhesion-resistant biodegradable side (14).

34. The biodegradable surgical implant (10) as set forth in claim 30, wherein:
the biodegradable surgical implant contains a single layer of resorbable polymer base material;
the tissue-ingrowth biodegradable side (12) is integrally formed with the resorbable polymer base material; and
the adhesion-resistant biodegradable side (14) is integrally formed with the resorbable polymer base material.

35. The biodegradable surgical implant (10) as set forth in claim 30, wherein the tissue-ingrowth biodegradable side (12) is formed to have an open, non-smooth and featured surface.

36. The biodegradable surgical implant (10) as set forth in claim 35, wherein the tissue-ingrowth biodegradable side (12) is formed to have one or more of alveoli and pores.

37. The biodegradable surgical implant (10) as set forth in claim 36, wherein the tissue-ingrowth biodegradable side (12) is formed to have one or more of alveoli and pores distributed irregularly on the tissue-ingrowth biodegradable side.

38. The biodegradable surgical implant (10) as set forth in claim 36, wherein the tissue-ingrowth biodegradable side (12) is formed to have pores, which are distributed In an Irregular fashion and which are visible to the naked eye.

39. The biodegradable surgical implant (10) as set forth in claim 35, wherein the tissue-ingrowth biodegradable side (12) is formed to have one or more of a cracked, broken or flaked surface, which causes tissue turbulence and inflammation between tissues of the host and the tissue-ingrowth biodegradable side (12).

40. The biodegradable surgical implant (10) as set forth in claim 1, wherein the adhesion-resistant biodegradable side (14) is constructed with one or more of (a) a surface topography and (2) a surface composition, which differs from that of the tissue-ingrowfh biodegradable side (12) and which facilitates, relative to the tissue-ingrowth biodegradable side (12), an anti-adhesive effect between the biodegradable surgical implant (10) and tissue of the host.

41. The biodegradable surgical implant (10) as set forth in claim 40, wherein the adhesion-resistant biodegradable side (14) is constructed with a surface topography, which differs from that of the tissue-ingrowth biodegradable side(12) and which facilitates, relative to the tissue-ingrowth biodegradable side(12), an anti-adhesive effect between the biodegradable surgical implant (10) and tissue of the host.

42. The biodegradable surgical implant (10) as set forth in claim 40, wherein the adhesion-resistant biodegradable side (14) comprises a substantially smooth surface.

43. The biodegradable surgical implant (10) as set forth in claim 40, wherein:
the adhesion-resistant biodegradable side (14) comprises a first layer of biodegradable material; and
the tissue-ingrowth biodegradable side (12) comprises a second layer of biodegradable material.

44. The biodegradable surgical implant (10) as set forth in claim 40, wherein the adhesion-resistant biodegradable side (14) comprises a first layer of nonporous biodegradable material; and
the tissue-ingrowth biodegradable side (12) comprises a second layer of nonporous biodegradable material.

## Patentansprüche

1. Biologisch abbaubares chirurgisches Implantat (10) zur Implantation in einem Wirt, umfassend eine im Wesentlichen planare Barrieremembran aus einem resorbierbaren Polymerbasismaterial mit
einer biologisch abbaubaren Seite zum Einwachsen von bzw. in Gewebe (12) und
einer adhäsionsbeständigen, biologisch abbaubaren Seite (14),
wobei die biologisch abbaubare Seite zum Einwachsen von bzw. in Gewebe (12) und die adhäsionsbeständige, biologisch abbaubare Seite (14) eine zweite bzw. eine erste Schicht eines biologisch abbaubaren Materials umfassen und sich in Bezug auf sowohl das Oberflächenaussehen als auch die Oberflächenfunktion unterscheiden,
**dadurch gekennzeichnet, dass** die biologisch abbaubare Seite zum Einwachsen von bzw. in Gewebe und die adhäsionsbeständige, biologisch abbaubare Seite ein nichtporöses, biologisch abbaubares Material umfassen.

2. Biologisch abbaubares chirurgisches Implantat (10) nach Anspruch 1, wobei die biologisch abbaubare Seite zum Einwachsen von bzw. in Gewebe (12) und die adhäsionsbeständige, biologisch abbaubare Seite (14) aus im Wesentlichen dem gleichen Material gebildet sind.

3. Biologisch abbaubares chirurgisches Implantat (10) nach Anspruch 2, wobei die biologisch abbaubare Seite zum Einwachsen von bzw. in Gewebe (12) oder die adhäsionsbeständige, biologisch abbaubare Seite (14) ein Additiv umfasst.

4. Biologisch abbaubares chirurgisches Implantat (10) nach Anspruch 3, wobei das Additiv die biologische Abbaurate der adhäsionsbeständigen, biologisch abbaubaren Seite (14) in Bezug auf die der biologisch abbaubaren Seite zum Einwachsen von bzw. in Gewebe (12) erhöht.

5. Biologisch abbaubares chirurgisches Implantat (10) nach Anspruch 3, wobei das Additiv die biologische Abbaurate der biologisch abbaubaren Seite zum Einwachsen von bzw. in Gewebe (12) in Bezug auf die der adhäsionsbeständigen, biologisch abbaubaren Seite (14) verringert.

6. Biologisch abbaubares chirurgisches Implantat (10) nach Anspruch 1, wobei die biologisch abbaubare Seite zum Einwachsen von bzw. in Gewebe (12) oder die adhäsionsbeständige, biologisch abbaubare Seite (14) eine erste Zusammensetzung umfasst, die, in Bezug auf eine zweite Zusammensetzung der anderen Seite der biologisch abbaubaren Seite zum Einwachsen von bzw. in Gewebe bzw. der adhäsionsbeständigen, biologisch abbaubaren Seite, die Festigkeit oder Strukturintegrität der Seite beeinflusst.

7. Biologisch abbaubares chirurgisches Implantat (10) nach Anspruch 6, wobei die erste Zusammensetzung eine erste Polymerzusammensetzung umfasst und die zweite Zusammensetzung eine zweite Polymerzusammensetzung, die von der ersten Polymerzusammensetzung verschieden ist, umfasst.

8. Biologisch abbaubares chirurgisches Implantat (10) nach Anspruch 6, wobei die erste Zusammensetzung der biologisch abbaubaren Seite zum Einwachsen von bzw. in Gewebe (12) eine größere Festigkeit verleiht als die Festigkeit, die die biologisch abbaubare Seite zum Einwachsen von bzw. in Gewebe (12) aufweisen würde, wenn sie aus der zweiten Zusammensetzung gebildet wäre.

9. Biologisch abbaubares chirurgisches Implantat (10) nach Anspruch 8, wobei die erste Zusammensetzung Festigungs- und Verstärkungsfasern umfasst.

10. Biologisch abbaubares chirurgisches Implantat (10) nach Anspruch 8, wobei die erste Zusammensetzung eine erste Polymerzusammensetzung umfasst und die zweite Zusammensetzung eine zweite Polymerzusammensetzung, die von der ersten Polymerzusammensetzung verschieden ist, umfasst.

11. Biologisch abbaubares chirurgisches Implantat (10) nach Anspruch 2, wobei:
die biologisch abbaubare Seite zum Einwachsen von bzw. in Gewebe (12) eine erste Zusammensetzung umfasst;
die adhäsionsbeständige, biologisch abbaubare Seite (14) eine zweite Zusammensetzung umfasst; und
die adhäsionsbeständige, biologisch abbaubare Seite (14) eine Adhäsionsbeständigkeit umfasst, die größer als die Adhäsionsbeständigkeit ist, die durch die adhäsionsbeständige, biologisch abbaubare Seite (14) erhalten würde, wenn diese aus der ersten Zusammensetzung gebildet wäre.

12. Biologisch abbaubares chirurgisches Implantat (10) nach Anspruch 11, wobei die erste Zusammensetzung eine erste Polymerzusammensetzung umfasst und die zweite Zusammensetzung eine zweite Polymerzusammensetzung, die von der ersten Polymerzusammensetzung verschieden ist, umfasst.

13. Biologisch abbaubares chirurgisches Implantat (10) nach Anspruch 1, wobei:
die biologisch abbaubare Seite zum Einwachsen von bzw. in Gewebe (12) eine erste Zusammensetzung umfasst;
die adhäsionsbeständige, biologisch abbaubare Seite (14) eine zweite Zusammensetzung umfasst; und
die adhäsionsbeständige, biologisch abbaubare Seite (14) eine Adhäsionsbeständigkeit umfasst, die größer als die Adhäsionsbeständigkeit ist, die durch die adhäsionsbeständige, biologisch abbaubare Seite (14) erhalten würde, wenn diese aus der ersten Zusammensetzung gebildet wäre.

14. Biologisch abbaubares chirurgisches Implantat (10) nach Anspruch 13, wobei die erste Zusammensetzung eine erste Polymerzusammensetzung umfasst und die zweite Zusammensetzung eine zweite Polymerzusammensetzung, die von der ersten Polymerzusammensetzung verschieden ist, umfasst.

15. Biologisch abbaubares chirurgisches Implantat (10) nach Anspruch 1, wobei:
das biologisch abbaubare chirurgische Implantat eine einzige Schicht aus einem resorbierbaren Polymerbasismaterial mit einer im Wesentlichen gleichförmigen Zusammensetzung enthält; und
die Dicke der einzigen Schicht aus einem resorbierbaren Polymerbasismaterial mehr als etwa 500 µm beträgt.

16. Biologisch abbaubares chirurgisches Implantat (10) nach Anspruch 15, wobei die einzige Schicht aus einem resorbierbaren Polymerbasismaterial ein Material umfasst, das aus der Gruppe von einem Polylactidpolymer und einem Copolymer von zwei oder mehreren Polylactiden ausgewählt ist.

17. Biologisch abbaubares chirurgisches Implantat (10) nach Anspruch 15, wobei das resorbierbare Polymerbasismaterial Poly(L-lactid-co-D,L-lactid) ist.

18. Vorrichtung nach Anspruch 15, wobei die einzige Schicht aus einem resorbierbaren Polymerbasismaterial nicht fluiddurchlässig ist.

19. Biologisch abbaubares chirurgisches Implantat (10) nach Anspruch 1, wobei nur eine Seite des biologisch abbaubaren chirurgischen Implantats mit mindestens einer Komponente von einem Anti-Bone-Mittel, einer chemotaktischen Substanz zur Beeinflussung der Zellmigration, einer Hemmsubstanz zur Beeinflussung der Zellmigration, einem mitogenen Wachstumsfaktor zur Beeinflussung der Zellproliferation, einem Wachstumsfaktor zur Beeinflussung der Zelldifferenzierung und Faktoren, die die Angiogenese fördern, getränkt ist.

20. Biologisch abbaubares chirurgisches Implantat (10) nach Anspruch 1, wobei das biologisch abbaubare chirurgische Implantat in einer sterilen Verpackung dicht verschlossen ist.

21. Biologisch abbaubares chirurgisches Implantat (10) nach Anspruch 1, wobei das biologisch abbaubare chirurgische Implantat Narbenbildung verhindert.

22. Biologisch abbaubares chirurgisches Implantat (10) nach Anspruch 1, wobei das biologisch abbaubare chirurgische Implantat Gewebeadhäsion verhindert.

23. Biologisch abbaubares chirurgisches Implantat (10) nach Anspruch 1, wobei das biologisch abbaubare chirurgische Implantat ein Polylactidpolymer und ein Copolymer von mindestens zwei Polylactiden umfasst.

24. Biologisch abbaubares chirurgisches Implantat (10) nach Anspruch 1, wobei das biologisch abbaubare chirurgische Implantat fluidundurchlässig ist.

25. Biologisch abbaubares chirurgisches Implantat (10) nach Anspruch 1, wobei die biologisch abbaubare Seite zum Einwachsen von bzw. in Gewebe (12) und die adhäsionsbeständige, biologisch abbaubare Seite (14) aus dem gleichen Material gebildet sind.

26. Biologisch abbaubares chirurgisches Implantat (10) nach Anspruch 1, wobei das biologisch abbaubare chirurgische Implantat eine einzige Schicht aus einem resorbierbaren Polymer enthält, die sowohl die biologisch abbaubare Seite zum Einwachsen von bzw. in Gewebe (12) als auch die adhäsionsbeständige, biologisch abbaubare Seite (14) bildet.

27. Biologisch abbaubares chirurgisches Implantat (10) nach Anspruch 1, wobei:
das biologisch abbaubare chirurgische Implantat eine einzige Schicht aus einem resorbierbaren Polymerbasismaterial enthält;
die biologisch abbaubare Seite zum Einwachsen von bzw. in Gewebe (12) integral mit dem resorbierbaren Polymerbasismaterial ausgebildet ist; und
die adhäsionsbeständige, biologisch abbaubare Seite (14) integral mit dem resorbierbaren Polymerbasismaterial ausgebildet ist.

28. Biologisch abbaubares chirurgisches Implantat (10) nach Anspruch 1, wobei die adhäsionsbeständige, biologisch abbaubare Seite (14) und die biologisch abbaubare Seite zum Einwachsen von bzw. in Gewebe (12) verschiedene Materialien umfassen.

29. Biologisch abbaubares chirurgisches Implantat (10) nach Anspruch 1, wobei die biologisch abbaubare Seite zum Einwachsen von bzw. in Gewebe (12) mit (a) einem Oberflächenaussehen in der Form einer Oberflächentopographie und/oder (b) einer Oberflächenfunktion in der Form einer Oberflächenzusammensetzung aufgebaut ist, die von der der gegen Adhäsion wirkenden, biologisch abbaubaren Seite verschieden ist und die die Festigkeit und/oder Langlebigkeit und/oder eine wesentliche Fibroblastenreaktion in Gewebe des Wirts gegenüber der adhäsionsbeständigen, biologisch abbaubaren Seite (14) erleichtert.

30. Biologisch abbaubares chirurgisches Implantat (10) nach Anspruch 29, wobei die biologisch abbaubare Seite zum Einwachsen von bzw. in Gewebe (12) mit einer Oberflächentopographie aufgebaut ist, die von der der Gegenadhäsion wirkenden biologisch abbaubaren Seite verschieden ist, und die Festigkeit und/oder Langlebigkeit und/oder eine wesentliche Fibroblastenreaktion in Gewebe des Wirts gegenüber der adhäsionsbeständigen, biologisch abbaubaren Seite (14) erleichtert.

31. Biologisch abbaubares chirurgisches Implantat (10) nach Anspruch 30, wobei die biologisch abbaubare Seite zum Einwachsen von bzw. in Gewebe (12) und die adhäsionsbeständige, biologisch abbaubare Seite (14) aus im Wesentlichen dem gleichen Material gebildet sind.

32. Biologisch abbaubares chirurgisches Implantat (10) nach Anspruch 30, wobei die biologisch abbaubare Seite zum Einwachsen von bzw. in Gewebe (12) und die adhäsionsbeständige, biologisch abbaubare Seite (14) aus dem gleichen Material gebildet sind.

33. Biologisch abbaubares chirurgisches Implantat (10) nach Anspruch 30, wobei das biologisch abbaubare chirurgische Implantat eine einzige Schicht aus einem resorbierbaren Polymer enthält, das sowohl die biologisch abbaubare Seite zum Einwachsen von bzw. in Gewebe (12) als auch die adhäsionsbeständige biologisch abbaubare Seite (14) bildet.

34. Biologisch abbaubares chirurgisches Implantat (10) nach Anspruch 30, wobei:
das biologisch abbaubare chirurgische Implantat eine einzige Schicht aus einem resorbierbaren Polymerbasismaterial enthält;
die biologisch abbaubare Seite zum Einwachsen von bzw. in Gewebe (12) integral mit dem resorbierbaren Polymerbasismaterial ausgebildet ist; und
die adhäsionsbeständige, biologisch abbaubare Seite (14) integral mit dem resorbierbaren Polymerbasismaterial ausgebildet ist.

35. Biologisch abbaubares chirurgisches Implantat (10) nach Anspruch 30, wobei die biologisch abbaubare Seite zum Einwachsen von bzw. in Gewebe (12) derart ausgebildet ist, dass sie eine offene, nicht glatte und charakteristische Merkmale zeigende Oberfläche aufweist.

36. Biologisch abbaubares chirurgisches Implantat (10) nach Anspruch 35, wobei die biologisch abbaubare Seite zum Einwachsen von bzw. in Gewebe (12) derart ausgebildet ist, dass sie Alveoli und/oder Poren aufweist.

37. Biologisch abbaubares chirurgisches Implantat (10) nach Anspruch 36, wobei die biologisch abbaubare Seite zum Einwachsen von bzw. in Gewebe (12) derart ausgebildet ist, dass sie Alveoli und/oder Poren aufweist, die unregelmäßig auf der biologisch abbaubaren Seite zum Einwachsen von bzw. in Gewebe verteilt sind.

38. Biologisch abbaubares chirurgisches Implantat (10) nach Anspruch 36, wobei die biologisch abbaubare Seite zum Einwachsen von bzw. in Gewebe (12) derart ausgebildet ist, dass sie Poren aufweist, die in unregelmäßiger Weise verteilt und für das bloße Auge sichtbar sind.

39. Biologisch abbaubares chirurgisches Implantat (10) nach Anspruch 35, wobei die biologisch abbaubare Seite zum Einwachsen von bzw. in Gewebe (12) derart ausgebildet ist, dass sie eine gerissene und/oder gebrochene und/oder geschuppte Oberfläche aufweist, wodurch Gewebeturbulenz und -entzündung zwischen Geweben des Wirts und der biologisch abbaubaren Seite zum Einwachsen von bzw. in Gewebe (12) verursacht werden.

40. Biologisch abbaubares chirurgisches Implantat (10) nach Anspruch 1, wobei die adhäsionsbeständige, biologisch abbaubare Seite (14) mit (a) einer Oberflächentopographie und/oder (b) einer Oberflächenzusammensetzung aufgebaut ist, die von der der biologisch abbaubaren Seite zum Einwachsen von bzw. in Gewebe (12) verschieden ist und die, gegenüber der biologisch abbaubaren Seite zum Einwachsen von bzw. in Gewebe (12), eine Antiadhäsionswirkung zwischen dem biologisch abbaubaren chirurgischen Implantat (10) und Gewebe des Wirts erleichtert.

41. Biologisch abbaubares chirurgisches Implantat (10) nach Anspruch 40, wobei die adhäsionsbeständige, biologisch abbaubare Seite (14) mit einer Oberflächentopographie aufgebaut ist, die von der der biologisch abbaubaren Seite zum Einwachsen von bzw. in Gewebe (12) verschieden ist und die, gegenüber der biologisch abbaubaren Seite zum Einwachsen von bzw. in Gewebe (12), eine Antiadhäsionswirkung zwischen dem biologisch abbaubaren chirurgischen Implantat (10) und Gewebe des Wirts erleichtert.

42. Biologisch abbaubares chirurgisches Implantat (10) nach Anspruch 40, wobei die adhäsionsbeständige, biologisch abbaubare Seite (14) eine im Wesentlichen glatte Oberfläche umfasst.

43. Biologisch abbaubares chirurgisches Implantat (10) nach Anspruch 40, wobei:
die adhäsionsbeständige, biologisch abbaubare Seite (14) eine erste Schicht aus einem biologisch abbaubaren Material umfasst; und
die biologisch abbaubare Seite zum Einwachsen von bzw. in Gewebe (12) eine zweite Schicht aus einem biologisch abbaubaren Material umfasst.

44. Biologisch abbaubares chirurgisches Implantat (10) nach Anspruch 40, wobei:
die adhäsionsbeständige, biologisch abbaubare Seite (14) eine erste Schicht aus einem nichtporösen, biologisch abbaubaren Material umfasst; und
die biologisch abbaubare Seite zum Einwachsen von bzw. in Gewebe (12) eine zweite Schicht aus einem nichtporösen, biologisch abbaubaren Material umfasst.

## Revendications

1. Implant chirurgical biodégradable (10) pour implantation dans un hôte, comprenant une membrane barrière sensiblement plane d'un matériau de base polymère résorbable ayant
un côté biodégradable à croissance interne de tissu (12) et
un côté biodégradable résistant à l'adhérence (14),
le côté biodégradable à croissance interne de tissu (12) et le côté biodégradable résistant à l'adhérence (14) comprenant une seconde et une première couche de matériau biodégradable, respectivement, et étant différents à la fois en aspect de surface et en fonction de surface, **caractérisé en ce que**
le côté biodégradable à croissance interne de tissu et le côté biodégradable résistant à l'adhérence comprennent un matériau biodégradable non poreux.

2. Implant chirurgical biodégradable (10) selon la revendication 1, dans lequel le côté biodégradable à croissance interne de tissu (12) et le côté biodégradable résistant à l'adhérence (14) sont formés sensiblement du même matériau.

3. Implant chirurgical biodégradable (10) selon la revendication 2, dans lequel l'un du côté biodégradable à croissance interne de tissu (12) et du côté biodégradable résistant à l'adhérence (14) comprend un additif.

4. Implant chirurgical biodégradable (10) selon la revendication 3, dans lequel l'additif accroit une vitesse de biodégradation du côté biodégradable résistant à l'adhérence (14) par rapport à celle du côté biodégradable à croissance interne de tissu (12).

5. Implant chirurgical biodégradable (10) selon la revendication 3, dans lequel l'additif réduit une vitesse de biodégradation du côté biodégradable à croissance interne de tissu (12) par rapport à celle du côté biodégradable résistant à l'adhérence (14).

6. Implant chirurgical biodégradable (10) selon la revendication 1, dans lequel l'un du côté biodégradable à croissance interne de tissu (12) et du côté biodégradable résistant à l'adhérence (14) comprend une première composition affectant la résistance mécanique ou l'intégrité structurelle du côté par rapport à une seconde composition de l'autre du côté biodégradable à croissance interne de tissu et du côté biodégradable résistant à l'adhérence.

7. Implant chirurgical biodégradable (10) selon la revendication 6, dans lequel la première composition comprend une première composition de polymère et la seconde composition comprend une seconde composition de polymère qui diffère de la première composition de polymère.

8. Implant chirurgical biodégradable (10) selon la revendication 6, dans lequel la première composition confère au côté biodégradable à croissance interne de tissu (12) une résistance mécanique plus grande qu'une résistance mécanique que le côté biodégradable à croissance interne de tissu (12) aurait s'il était formé de la seconde composition.

9. Implant chirurgical biodégradable (10) selon la revendication 8, dans lequel la première composition comprend des fibres de rigidification et de renfort.

10. Implant chirurgical biodégradable (10) selon la revendication 8, dans lequel la première composition comprend une première composition de polymère et la seconde composition comprend une seconde composition de polymère qui est différente de la première composition de polymère.

11. Implant chirurgical biodégradable (10) selon la revendication 2, dans lequel:
le côté biodégradable à croissance interne de tissu (12) comprend une première composition ;
le côté biodégradable résistant à l'adhérence (14) comprend une seconde composition ; et
le côté biodégradable résistant à l'adhérence (14) comprend une résistance à l'adhérence qui est plus grande qu'une résistance à l'adhérence qui serait conférée par le côté biodégradable résistant à l'adhérence (14) s'il était formé de la première composition.

12. Implant chirurgical biodégradable (10) selon la revendication 11, dans lequel la première composition comprend une première composition de polymère et la seconde composition comprend une seconde composition de polymère qui est différente de la première composition de polymère.

13. Implant chirurgical biodégradable (10) selon la revendication 1, dans lequel :
le côté biodégradable à croissance interne de tissu (12) comprend une première composition ;
le côté biodégradable résistant à l'adhérence (14) comprend une seconde composition ; et
le côté biodégradable résistant à l'adhérence (14) comprend une résistance à l'adhérence qui est plus grande qu'une résistance à l'adhérence qui serait conférée par le côté biodégradable résistant à l'adhérence (14) s'il était formé de la première composition.

14. Implant chirurgical biodégradable (10) selon la revendication 13, dans lequel la première composition comprend une première composition de polymère et la seconde composition comprend une seconde composition de polymère qui est différente de la première composition de polymère.

15. Implant chirurgical biodégradable (10) selon la revendication 1, dans lequel :
l'implant chirurgical biodégradable comprend une monocouche de matériau de base polymère résorbable ayant une composition sensiblement uniforme ; et
une épaisseur de la monocouche de matériau de base polymère résorbable est supérieure à environ 500 microns.

16. Implant chirurgical biodégradable (10) selon la revendication 15, dans lequel la monocouche de matériau de base polymère résorbable comprend un matériau choisi dans le groupe constitué par un polymère poly(lactide) et un copolymère de deux poly(lactides) ou plus.

17. Implant chirurgical biodégradable (10) selon la revendication 15, dans lequel le matériau de base polymère résorbable est le poly(L-lactide-co-D-L-lactide).

18. Dispositif selon la revendication 15, dans lequel la monocouche de matériau de base polymère résorbable n'est pas perméables aux fluides.

19. Implant chirurgical biodégradable (10) selon la revendication 1, dans lequel seul un côté de l'implant chirurgical biodégradable est imprégné avec au moins l'un parmi un agent anti-osseux, une substance chimiotactique permettant d'influencer la migration cellulaire, une substance inhibitrice permettant d'influencer la migration cellulaire, un facteur à croissance mitogène permettant d'influencer la prolifération cellulaire, un facteur à croissance permettant d'influencer la différenciation cellulaire et des facteurs qui favorisent l'angiogenèse.

20. Implant chirurgical biodégradable (10) selon la revendication 1, dans lequel l'implant chirurgical biodégradable est scellé dans un emballage stérile.

21. Implant chirurgical biodégradable (10) selon la revendication 1, dans lequel l'implant chirurgical biodégradable prévient la formation de cicatrices.

22. Implant chirurgical biodégradable (10) selon la revendication 1, dans lequel l'implant chirurgical biodégradable prévient l'adhérence de tissus.

23. Implant chirurgical biodégradable (10) selon la revendication 1, dans lequel l'implant chirurgical biodégradable comprend un polymère poly(lactide) et un copolymère d'au moins deux poly(lactides).

24. Implant chirurgical biodégradable (10) selon la revendication 1, dans lequel l'implant chirurgical biodégradable est imperméable aux fluides.

25. Implant chirurgical biodégradable (10) selon la revendication 1, dans lequel le côté biodégradable à croissance interne de tissu (12) et le côté biodégradable résistant à l'adhérence (14) sont formés du même matériau.

26. Implant chirurgical biodégradable (10) selon la revendication 1, dans lequel l'implant chirurgical biodégradable comprend une monocouche de polymère résorbable qui forme à la fois le côté biodégradable à croissance interne de tissu (12) et le côté biodégradable résistant à l'adhérence (14).

27. Implant chirurgical biodégradable (10) selon la revendication 1, dans lequel :
l'implant chirurgical biodégradable contient une monocouche de matériau de base polymère résorbable ;
le côté biodégradable à croissance interne de tissu (12) est formé solidairement avec le matériau de base polymère résorbable ; et
le côté biodégradable résistant à l'adhérence (14) est formé solidairement avec le matériau de base polymère résorbable.

28. Implant chirurgical biodégradable (10) selon la revendication 1, dans lequel le côté biodégradable résistant à l'adhérence (14) et le côté biodégradable à croissance interne de tissu (12) comprennent des matériaux différents.

29. Implant chirurgical biodégradable (10) selon la revendication 1, dans lequel le côté biodégradable à croissance interne de tissu (12) est construit avec un ou plusieurs éléments parmi (a) un aspect de surface sous la forme d'une topographie de surface et (2) une fonction de surface sous la forme d'une composition de surface, qui diffère de celle du côté biodégradable anti-adhérence et qui facilite un ou plusieurs éléments parmi la résistance mécanique, la longévité et une réaction fibroblastique substantielle dans le tissu de l'hôte par rapport au côté biodégradable résistant à l'adhérence (14).

30. Implant chirurgical biodégradable (10) selon la revendication 29, dans lequel le côté biodégradable à croissance interne de tissu (12) est construit avec une topographie de surface, qui diffère de celle du côté biodégradable anti-adhérence et qui facilite un ou plusieurs éléments parmi la résistance mécanique, la longévité et une réaction fibroblastique substantielle dans un tissu de l'hôte par rapport au côté biodégradable résistant à l'adhérence (14).

31. Implant chirurgical biodégradable (10) selon la revendication 30, dans lequel le côté biodégradable à croissance interne de tissu (12) et le côté biodégradable résistant à l'adhérence (14) sont formés sensiblement du même matériau.

32. Implant chirurgical biodégradable (10) selon la revendication 30, dans lequel le côté biodégradable à croissance interne de tissu (12) et le côté biodégradable résistant à l'adhérence (14) sont formés du même matériau.

33. Implant chirurgical biodégradable (10) selon la revendication 30, dans lequel l'implant chirurgical biodégradable contient une monocouche de polymère résorbable qui forme à la fois le côté biodégradable à croissance interne de tissu (12) et le côté biodégradable résistant à l'adhérence (14).

34. Implant chirurgical biodégradable (10) selon la revendication 30, dans lequel :
l'implant chirurgical biodégradable contient une monocouche de matériau de base polymère résorbable ;
le côté biodégradable à croissance interne de tissu (12) est formé solidairement avec le matériau de base polymère résorbable ; et
le côté biodégradable résistant à l'adhérence (14) est formé solidairement avec le matériau de base polymère résorbable.

35. Implant chirurgical biodégradable (10) selon la revendication 30, dans lequel le côté biodégradable à croissance interne de tissu (12) est formé pour avoir une surface ouverte, non lisse et caractéristique.

36. Implant chirurgical biodégradable (10) selon la revendication 35, dans lequel le côté biodégradable à croissance interne de tissu (12) est formé pour avoir un ou plusieurs éléments parmi des alvéoles et des pores.

37. Implant chirurgical biodégradable (10) selon la revendication 36, dans lequel le côté biodégradable à croissance interne de tissu (12) est formé pour avoir un ou plusieurs éléments parmi des alvéoles et des pores distribués irrégulièrement sur le côté biodégradable à croissance interne de tissu.

38. Implant chirurgical biodégradable (10) selon la revendication 36, dans lequel le côté biodégradable à croissance interne de tissu (12) est formé pour avoir des pores, qui sont distribués de manière irrégulière et qui sont visibles à l'oeil nu.

39. Implant chirurgical biodégradable (10) selon la revendication 35, dans lequel le côté biodégradable à croissance interne de tissu (12) est formé pour avoir un ou plusieurs éléments parmi une surface fissurée, rompue ou floconneuse, qui entraîne une turbulence et une inflammation du tissu entre les tissus de l'hôte et le côté biodégradable à croissance interne de tissu (12).

40. Implant chirurgical biodégradable (10) selon la revendication 1, dans lequel le côté biodégradable résistant à l'adhérence (14) est construit avec un ou plusieurs éléments parmi (a) une topographie de surface et (2) une composition de surface, qui diffère de celle du côté biodégradable à croissance interne de tissu (12) et qui facilite, par rapport au côté biodégradable à croissance interne de tissu (12), un effet anti-adhérent entre l'implant chirurgical biodégradable (10) et le tissu de l'hôte.

41. Implant chirurgical biodégradable (10) selon la revendication 40, dans lequel le côté biodégradable résistant à l'adhérence (14) est construit avec une topographie de surface, qui diffère de celle du côté biodégradable à croissance interne de tissu (12) et qui facilite, par rapport au côté biodégradable à croissance interne de tissu (12), un effet anti-adhérent entre l'implant chirurgical biodégradable (10) et le tissu de l'hôte.

42. Implant chirurgical biodégradable (10) selon la revendication 40, dans lequel le côté biodégradable résistant à l'adhérence (14) comprend une surface sensiblement lisse.

43. Implant chirurgical biodégradable (10) selon la revendication 40, dans lequel :
le côté biodégradable résistant à l'adhérence (14) comprend une première couche de matériau biodégradable ;
le côté biodégradable à croissance interne de tissu (12) comprend une seconde couche de matériau biodégradable.

44. Implant chirurgical biodégradable (10) selon la revendication 40, dans lequel le côté biodégradable résistant à l'adhérence (14) comprend une première couche de matériau biodégradable non poreux ; et
le côté biodégradable à croissance interne de tissu (12) comprend une seconde couche de matériau biodégradable non poreux.
